# EUROPEAN PATENT SPECIFICATION

(11) **EP 1 618 477 B1**
(45) Date of publication and mention of the grant of the patent: **07.10.2020**
(21) Application number: 04750952.6
(22) Date of filing: 29.04.2004
(51) Int. Cl.: H04L 29/06, G06F 21/62

(54) **EXTRANET SERVICE SITE AND METHOD FOR USING SAME**
EXTRANET-DIENST-SITE UND VERFAHREN ZU IHRER VERWENDUNG
SITE DE SERVICES EXTRANET ET PROCEDE POUR L'UTILISER

(30) Priority: 30.04.2003 US 466434 P; 30.04.2003 US 466439 P
(43) Date of publication of application: 25.01.2006
(73) Proprietor: Becton, Dickinson and Company, Franklin Lakes, NJ 07417-1880 (US)
(72) Inventor: HARPER, Patricia, Saddle Brook, New Jersey 07663 (US); DUBROWNY, Nancy, Garfield, New Jersey 07026 (US); BUTLER, Michele, Saddle Brook, New Jersey 07663 (US); GUNTER, James, Raleigh, North Carolina 27612 (US)
(74) Representative: dompatent von Kreisler Selting Werner - Partnerschaft von Patent- und Rechtsanwälten mbB
(86) International application number: PCT/US2004/013321
(87) International publication number: WO 2004/099914

(56) References cited:
- WO-A1-00/19324
- US-A1- 2002 123 898
- US-A1- 2004 050 929
- US-B1- 6 453 348
- US-B1- 6 799 177
- US-B2- 6 765 591
- PAKSTAS A: "Towards electronic commerce via science park multi-Extranets" COMPUTER COMMUNICATIONS, ELSEVIER SCIENCE PUBLISHERS BV, AMSTERDAM, NL LNKD- DOI:10.1016/S0140-3664(99)00132-2, vol. 22, no. 14, 15 September 1999 (1999-09-15), pages 1351-1363, XP004179336 ISSN: 0140-3664
- OLIVER R W ED - NUNAMAKER J F JR ET AL: "Corporate policies for electronic commerce" SYSTEM SCIENCES, 1997, PROCEEDINGS OF THE THIRTIETH HWAII INTERNATIONA L CONFERENCE ON WAILEA, HI, USA 7-10 JAN. 1997, LOS ALAMITOS, CA, USA,IEEE COMPUT. SOC, US LNKD- DOI:10.1109/HICSS.1997.663396, vol. 4, 7 January 1997 (1997-01-07), pages 254-264, XP010271870 ISBN: 978-0-8186-7743-4

## Description

### Field of the Invention

The present invention relates to an access-restricted extranet product information exchange system, and more particularly, to providing and managing a multi-level extranet product information service site, wherein each successive level contains advanced product information and algorithms, such as information regarding the development of plastic blood collection tubes, or the conversion from glass to plastic blood collection tubes, and wherein access to each level is restricted based upon a predetermined permission scheme. This application claims priority under 35 U.S.C. §119(e) from a U.S. Provisional Patent Application of Patricia Harper et al., entitled "Extranet Service Site And Method For Using Same", Serial No. 60/466,439, filed on April 30, 2003, and from a U.S. Provisional Patent Application of Sol Green, entitled "Method And System For Validating Changes In Medical Practice, Procedures, And Product Choice", Serial No. 60/466,434, filed April 30, 2003. Related subject matter is also disclosed in a U.S. Patent Application of Scott Pardo et al., entitled "System And Method For Determining Clinical Equivalence Of Test Methods", Serial No. 10/096,102, filed on March 13, 2002.

### Background of the Invention

An important use of computers is the transfer of information over a network, and currently, the largest computer network in existence is the Internet. As discussed in U.S. Patent No. 6,370,573 issued to Bowman-Amuah, the Internet is a worldwide interconnection of computer networks that communicate using a common protocol. Millions of computers, from low-end personal computers to high-end super computers, are coupled to the Internet.

As known to those skilled in the art, the Internet grew out of work funded in the 1960s by the U.S. Defense Department's Advanced Research Projects Agency, and for some time was used primarily by researchers in universities and national laboratories to share information. As the existence of the Internet became more widely known, many users outside of the academic and research community (e.g., employees of large corporations) started to use the Internet to carry electronic mail.

In 1989, a new type of information system known as the World Wide Web ("the Web") was introduced to the Internet. Early development of the Web took place at CERN, the European Particle Physics Laboratory. The Web is a wide-area hypermedia information retrieval system constructed to provide wide access to a large universe of documents and information. At that time however, the Web was known to and used by the academic and research community only, as there was no easily available tool which allowed a technically untrained person to access the Web.

In 1993, researchers at the National Center for Supercomputing Applications (NCSA) released a Web browser called "Mosaic" that implemented a graphical user interface (GUI). Mosaic's graphical user interface was simple to learn, yet powerful, in that the Mosaic browser allowed a user to retrieve documents from the World Wide Web using simple point-and-click commands. Because the user did not have to be technically trained and the browser was relatively simple to use, it opened up the Internet to the masses.

The architecture of the Web follows a conventional client-server model. The terms "client" and "server" are used to refer to a computer's general role as a requester of data (i.e., the client) or provider of data (i.e., the server). Within the typical Web environment, Web browsers reside in clients and Web documents reside in servers, and the clients and servers communicate using a protocol called "HyperText Transfer Protocol" (HTTP). Locations within the Web environment are defined as "sites", and each typically includes a standardized uniform resource locator (URL) that identifies the site. A browser is used to open a connection to a server, or site, and initiate a request for a document. The server delivers the requested document, typically in the form of a text document coded in a standard Hypertext Markup Language (HTML) format. When the connection is closed in the above interaction, the server serves a passive role, i.e., it accepts commands from the client and cannot request the client to perform any action.

The communication model of the conventional Web environment provides a very limited level of interaction between clients and servers. In many systems, increasing the level of interaction between components in the system often makes the system more useful, but also increases its complexity and typically slows the rate of the interaction. Thus, the conventional Web environment architecture is configured to provide less complex, but faster interactions through implementation of a lower level of interaction between clients and servers.

However, developments in levels of interaction have led to the creation of an "extranet", which essentially refers to an intranet that is partially accessible to authorized outsiders. As known to those skilled in the art, an intranet is an organization's own network based on TCP/IP protocols, typically accessible only by the organization's members. Whereas an intranet resides behind a firewall and is accessible only to people who are members of the same company or organization, an extranet provides various levels of accessibility to outsiders. In such applications, access to an extranet typically requires a valid username and password, and an associated identity then determines which parts of the extranet a user can access. The following Table 1, presented by Kerstin Forsberg in a publication entitled "Extranet - A Reference Page", January 12, 2000, illustrates the commonly provided areas in each of the three interaction techniques described above.

**Table 1**

| | Internet | Intranet | Extranet |
|---|---|---|---|
| Access | Public | Private | Semi-private |
| Users | Everyone | Members of a specific firm | Group of closely related firms |
| Information | Fragmented | Proprietary | Shared in closely trusted held circles |

As discussed in U.S. Patent No. 6,453,348 issued to Barnier et al., many extranets allow communications and application sharing between designated, non-related organizations and consist of various architectures to facilitate these functions. One type of extranet architecture discussed in the Barnier et al. patent is often referred to as a shared private network, where the organizations that have access to the network are enumerated and often a third party is in charge of updating the list of users and managing user passwords. This type of shared private network between two organizations, for example, a shipping dock and receiving dock, can be linked to exchange information regarding orders. This is generally the oldest type of extranet. Such extranets have also been used for electronic data interchange (EDI) and involve coupling two organizations through a third party provider typically using X0.25 protocols and not necessarily using Internet Protocols ("IP").

Another type of extranet architecture discussed in the Barnier et al. patent couples a plurality of non-related organizations directly together with appropriate routing and traffic management capabilities. The organizations can communicate with one another and share various applications, however, some implementations are managed by one business partner.

Still other types of extranets utilize the Internet, which provides increased bandwidth but lacks the security of shared private networks. One such type of network which couples organizations through the Internet is referred to as a virtual private network (VPN). The virtual private network extranet utilizes the facilities of one or more Internet service providers combined with the user's own VPN software instead of setting up a dedicated private network. A main difference between a virtual private network extranet and the Internet, in general, is that in a virtual private network extranet access is restricted and limited to designated organizations and/or individuals rather than the world at large.

As the use of extranet sites can be restricted and limited, such sites become an effective tool for sharing proprietary information with users, such as product customers. In many cases, very current product information or algorithms tailored to answer a customer's specific needs in an area of research or development can be provided safely and securely in a user-friendly format via the secured extranet site. For example, very often in clinical testing a need exists to demonstrate clinical validation, which requires a clinically sound strategy including the latest information. In a specific example regarding the development of plastic blood collection tubes or the conversion from glass to plastic blood collection tubes, a user can require extensive information to determine specific analytes and instruments to test.

Accordingly, a need exists for a device and method to provide a network extranet site to share information between product manufacturers, developers and customers, including proprietary manufacturer information, for user access and use. The information can be restricted and protected such as through multi-level access based upon user access permission.

WO 00/19324 A1 discloses a system and method for providing a trusted server which controls access to the execution of processes by applying file level extended sensitivity label attributes.

A. Pakstas, "Towards electronic commerce via science park multi-Extranets" (Computer Communications 22, 1999, pages 1351 - 1363) discusses several extranet architectures.

### Summary of the Invention

It is therefore an object of the present invention to provide a system and method for a multi-level information and service database which can be accessed by remote users (e.g. product manufacturers, developers and customers), based upon a degree of access permission, service level agreement and/or operations level agreement between the site provider and each user, wherein such access allows the exchange of information and/or an application of one or more specific management operations and algorithms.

It is another object of the present invention to provide information and services in a multi-level form, wherein at each level a plurality of information databases, management operations and algorithms regarding the development of products, such as plastic blood collection tubes, or the conversion from glass to plastic blood collection tubes, can be performed as directed by the user.

These and other objects are substantially achieved in embodiments of the present invention by providing and managing a multi-level extranet product service site wherein each progressive level contains advanced product and customer use information, and access to each layer is restricted based upon a permission scheme. The information databases can be cumulative and can include various white papers, articles and publications of use by the user in product calculations, and further include algorithms for providing values in product calculations. The databases can be presented to a user via an extranet site to effectively provide user support regarding rapidly developing products, such as glass and plastic evacuated blood collection tubes for most, if not all, analytes evaluated in the clinical laboratory setting.

The subject matter of the invention is defined by each of independent claims 1 and 12.

### Brief Description of the Drawings

The above and other objects and advantages will be apparent upon consideration of the following drawings and detailed description. The preferred embodiments of the present invention are illustrated in the appended drawings in which like reference numerals refer to like elements and in which:
Fig. 1 is a block diagram of a conventional extranet architecture;
Fig. 2A is a flow chart illustrating a first embodiment of the present invention implemented at an extranet site;
Fig. 2B is an exemplary Analytical Array Spreadsheet of an access level in accordance with an embodiment of the present invention;
Fig. 3 is an exemplary screen display for directing access in accordance with an embodiment of the present invention; and
Fig. 4 is a flow chart illustrating a second embodiment of the present invention implemented at an extranet site.

In the drawing figures, it will be understood that like numerals refer to like structures.

### Detailed Description of the Preferred Embodiments

The embodiments of the present invention disclosed herein provide a multi-level extranet product service site wherein each progressive level contains advanced product and customer use information, and access to each layer is restricted based upon a permission scheme. The information databases can be cumulative and can include various white papers, articles and publications of use by the user in product calculations, and further include algorithms for providing values in product calculations

As described in greater detail below, the embodiments of the present invention include an extranet site which provides user support in the form of clinical data regarding rapidly developing products, such as glass and plastic evacuated blood collection tubes for most, if not all, analytes evaluated in the clinical laboratory setting. The data obtained via the present invention can be used to provide compliance support to clinical laboratory customers or site users that may be developing or converting a product, such as converting from glass to plastic blood collection tubes for collected blood samples. In the example described below, the present invention is used to provide support to users pursuing conversion from glass to plastic blood collection tubes; however, any number of compliance support programs or other applications can be implemented through the practice of the present invention.

The supporting data provided at the extranet site can include information provided from sources such as manufacturer data, customer white papers and third party sources, such as published journal articles. Such white papers and articles can also include customer communications containing clinical and technical data. Remaining additional data can further include external data shared by customers that have previously implemented product evaluations and that are willing to share or provide information on a limited or restricted basis with the manufacturer or other customers under one or more agreements. In the example presented below, this information can be provided by customers currently using plastic blood collection tubes, or those which have recently converted from glass to plastic blood collection tubes. In the present invention, customers can also be granted access to the proprietary information of major manufacturers, such as manufacturer databases and software at the extranet site upon signing a confidentiality agreement.

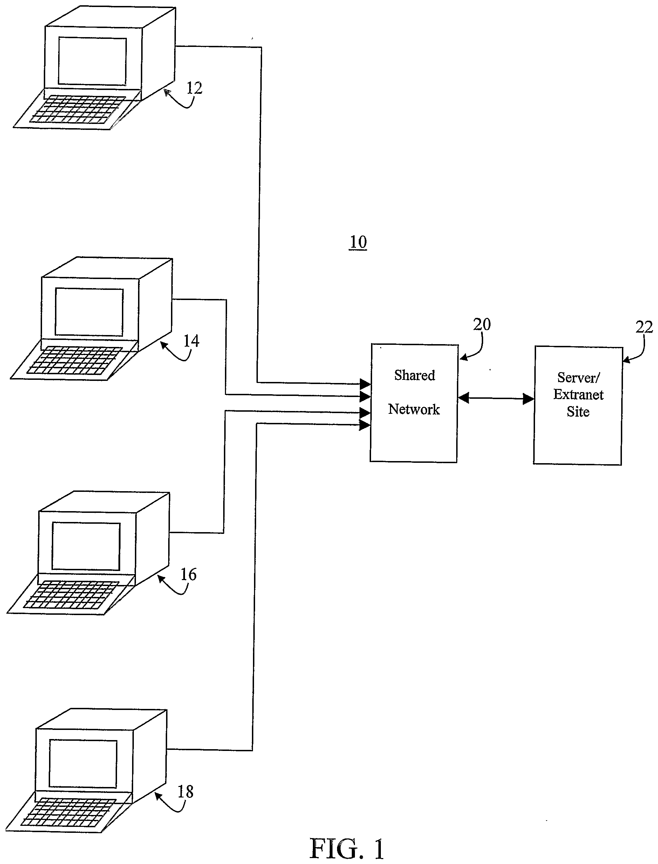

An exemplary extranet site constructed as a shared private network is shown in Fig. 1, wherein the users that have access to the network are enumerated and a third party is in charge of updating the list of users and managing user passwords. However, in other applications, the extranet site can be constructed by coupling two users through the third party provider using X0.25 protocols and not necessarily using Internet Protocols. In still other applications, the extranet site can be provided to couple a number of non-related users directly together with appropriate routing and traffic management capabilities. In this case, the users can communicate directly with one another and share various applications, wherein some applications are managed by one user or provider. Still other applications can include a virtual private network extranet that utilizes the facilities of one or more Internet service providers combined with the users' own VPN software instead of setting up a dedicated private or shared network. As noted above, a main difference between the shared network extranet and the Internet, in general, is that in the shared network extranet, access is restricted and limited to designated organizations and/or users rather than the world at large.

The architecture 10 of Fig. 1 includes a plurality of remote users 12, 14, 16 and 18, a shared network 20 utilizing Internet Protocol (IP), and a server 22. The shared network 20 couples the plurality of remote users to the server 22, which includes an interconnected grouping of storage devices and processors that stores a number of applications and databases for use. The remote users 12, 14, 16 and 18 can include stand-alone computer workstations as shown in Fig. 1, or can include wireless handheld devices such as Handspring Visor™ devices or other types of personal digital assistants (PDAs) located at one or more places.

In Fig. 1, the remote users, or subscribers 12, 14 and 16 can be non-related entities, for example, different companies or individuals, and a subscriber on the shared network 20 can have access to some or all of the applications and data available at the extranet site on the server 22 through their network connection. Access for an individual user can be determined by what applications the user or user's organization has permission to access, and can be achieved via a user's browser or through automatic distributions and updates to a user application. Typically however, Web browser technology is used to present each subscribing user with a menu of applications from which they can choose merely by clicking on text or a particular icon displayed on a display unit.

In Fig. 1, each user 12, 14, 16 and 18 can access the extranet architecture through a router using various options such as analog or ISDN dial-up access, or dedicated access using ATM, SMDS or frame relay protocols. Security can be provided in various ways such as by a firewall, passwords, encryption programs, digital certificates and user application security. The server 22 can also include at least one application server and a plurality of data storage units coupled together and to the firewall by at least one hub and a local area network. The number of data storage units can depend upon the number of applications provided and amount of user data stored on the server.

In the embodiment of the present invention described below, access to the shared network 20 can be controlled in a number of ways, such as through the use of confidentiality agreements between the user and extranet site provider, or confidentiality agreements between the user and one or more parties which provide information to the extranet site and/or other users. Specifically, a user can arrange a confidentiality agreement with the extranet site provider and gain access to one or more levels using a password or encryption/authorization key. In another application of the present invention where information at the extranet site is provided by a third party on a restricted basis, a user can arrange a confidentiality agreement with the extranet site provider and/or the third party and, thereafter, gain access to one or more levels including information provided by the third party. In doing so, the most current information from the third party is provided and access control is reserved by third party agreements.

Once access to the extranet site is allowed, the applications and data available on the server 22 are separated into multiple levels, wherein each level is accessible to remote individual users based upon access permission. The applications and data available at the extranet site on the server 22 is divided into separate levels based upon the amount and degree of restricted, private, or proprietary information contained therein, and additional user access permission can be granted by levels as described below.

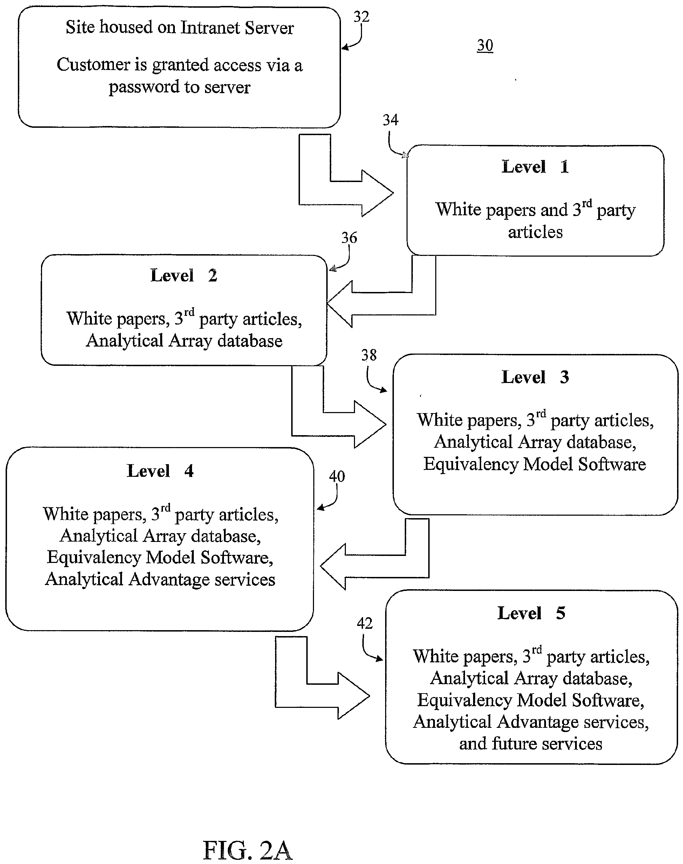

In a first embodiment of the present invention shown in Fig. 2A, an extranet site 30 provides at least five separate levels of current product information and/or algorithms tailored to answer a customer's specific needs in an area of research or development. In the example shown in Fig. 2A, the product information and/or algorithms are directed toward the development of plastic blood collection tubes or the conversion from glass to plastic blood collection tubes by a user. In the first embodiment, each level has a cumulative content from level one to level five. That is, each subsequent level includes new information, as well as all information disclosed in the earlier levels. Additional levels (e.g., added levels 6, 7, 8 and so forth) can also be provided, or the information provided on each level can be expanded (e.g., added sub-levels 1-A, 1-B, 1-C and so forth) as it becomes available or as required for effective user results.

In Fig. 2A, access to the extranet site is controlled at 32 via a security mechanism, such as a password provided via an access screen. The access screen can be used to identify a user or customer, and also to point the user to any updates based upon past searches and operations. The access screen can be further tailored as required by the user's past, present or anticipated future needs. Upon successful access, the user is taken to the level to which access permission is granted. In the first embodiment shown in Fig. 2A, each level is cumulative; therefore, the user gains access to the complete range of information for a given level, in addition to the information of preceding levels.

Level one, shown at 34 of Fig. 2A, can include white papers, third party articles, and any additional related materials regarding the development of or conversion to plastic blood collection tubes for collected blood samples. The information of level one is configured to be provided at a very low level of user authorization.

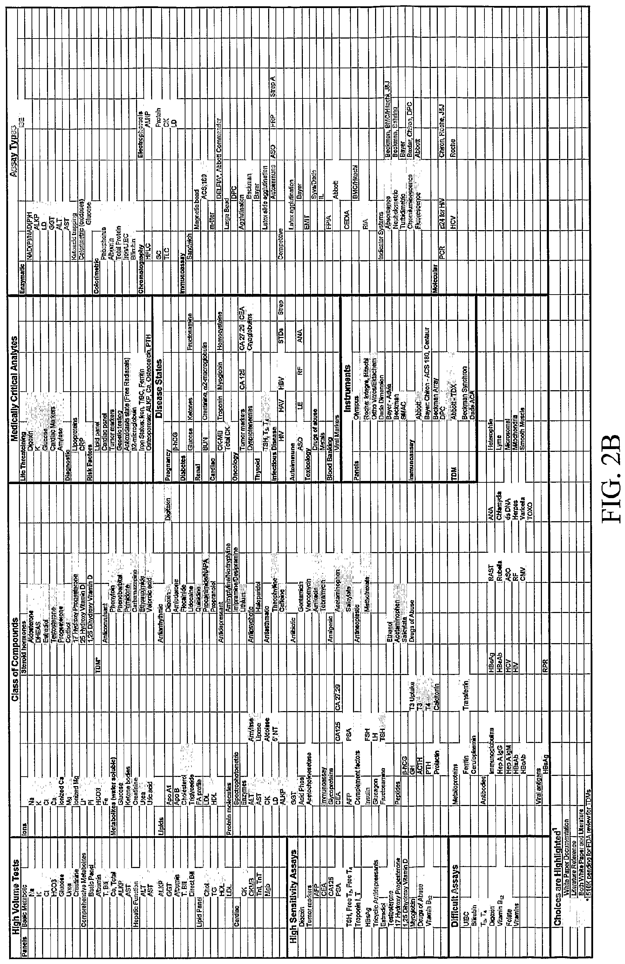

As each advanced layer is cumulative, level two, shown at 36, can include the contents of level one, plus an Analytical Array database regarding the development of or conversion to plastic blood collection tubes. One example of an Analytical Array database, shown in Fig. 2B, includes a LotusNotes database developed to include instruments and methodology for a large number of laboratory analytes that can be utilized by manufacturers to determine a testing matrix for blood collection tube product development. The database includes information regarding compounds, instruments, tests and assays, and subtopics within each can be provided as a highlighted selection. Such highlighting, or colored shading, can indicate the documentation available (i.e., white paper documentation).

Level three, shown at 38, can include the contents of levels one and two, plus Equivalency Model Software (EMS) provided to analyze clinical data by performing data comparisons, graphing the analysis, and indicating the clinical acceptability and/or equivalency of the data when developing or converting to plastic blood collection tubes. Additional details of exemplary equivalency model software are discussed in U.S. Patent Application Serial No. 10/096,102, referenced above.

Level four, shown at 40, can include the contents of levels one, two and three, plus Analytic Advantage services and related links, such as laboratory accreditation programs regarding the development or conversion of plastic blood collection tubes. These services can include internet links to related search engines, publications, instrument companies and laboratory societies. Level five, shown at 42, can include the contents of levels one through four, plus future and ongoing applications regarding the development or conversion of plastic blood collection tubes and would be provided at a very high level of user authorization.

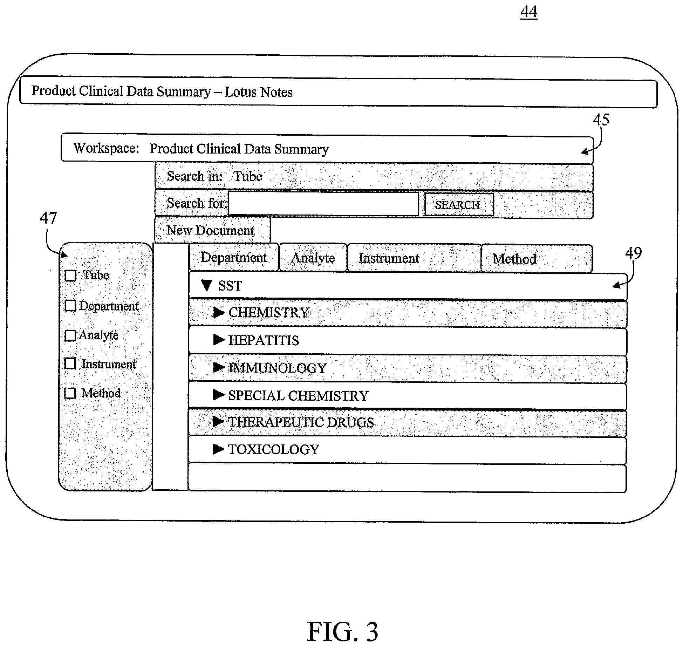

In the embodiment shown in Fig. 2A, each level is presented to the user as a screen menu. In Fig. 3, an example is shown of a screen menu that can be displayed on a remote user's computer in accordance with the first embodiment of the present invention.

A computer display example 44 is illustrated in Fig. 3 and includes various menus that represent information and operations available to the user at level one, and from which the user can select merely by selecting a topic. As noted above, level one can include white papers, third party journal published articles, and any additional related materials regarding the development of or conversion to plastic blood collection tubes, which can be provided at a very low level of user authorization. Although selections can be developed to accurately indicate the data or operation involved, in applications where an icon is used or a selection is unclear or presented in an unfamiliar language, a language translation feature can be included to provide universal user accessibility.

In block 45 along the top side of the display example 44, a workspace notation is provided to select and indicate the workspace being displayed. This can include white papers, articles, and related materials such as clinical data summaries regarding plastic blood collection tubes.

In block 47 along the left side of the display example 44, a list of categories for the selected workspace 45 are shown, including specifics for tests, analytes and instruments.

In block 49 along the right side of the display example 44, a list of topics for the selected category 47 for the selected workspace 45 are shown. These are topics for the selected category and can include narrow issues regarding clinical chemistry, tube preference, instrumentation, analyte and method of analysis. For each clinical laboratory discipline, hematology, coagulation, urinalysis, immunology, immunohematology and microbiology, additional pages can be created with identical topics identified for further confirmation.

The Lotus Notes application shown in Fig. 2B and the use of related data and operations shown in Fig. 3 are intended to be illustrative and not limiting. It will be appreciated that future levels can replace or eliminate topics illustrated while adding still new ones. Based upon the content at each level and associated access permission, additional lists, data or even icons (not shown) can be provided that represent multiple tasks, such as Java-based office applications which provide word processor, spreadsheet presentations and database applications. Another icon can be provided to represent a collaboration suite application which includes project management, discussion groups, work flow engines, document and image libraries and bulletin board applications. Yet another icon can be provided to represent an electronic data interchange (EDI) application which would enable low-volume EDI users to enter transactions into a Web page or enable medium-volume users to send transactions by secure EDI-MIME type e-mail. And still another icon can be provided to represent a Java applet vault application to provide, store and verify digital certificates for Java applets shared by subscribers.

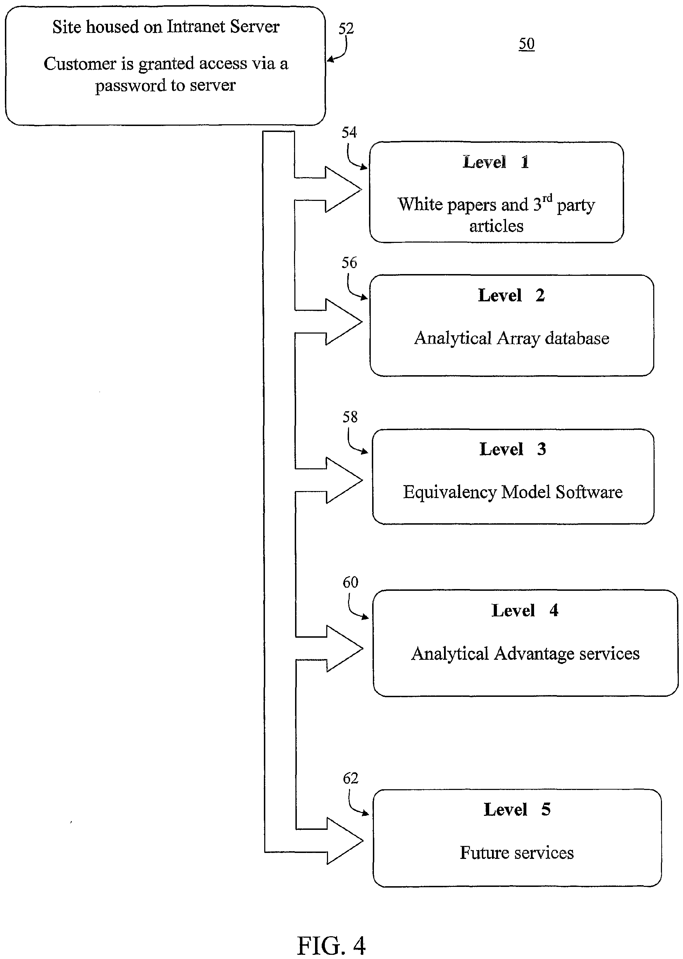

In another embodiment of the present invention shown in Fig. 4, the extranet site 50 includes five separate levels having non-cumulative content for each level, from level one to level five. That is, each subsequent level includes new information and excludes information disclosed in earlier or subsequent levels. In Fig. 4, access to the extranet site is controlled at 52 as described above, however, upon successful access, the user is taken to the level to which access permission is granted, and data present on all remaining levels is restricted. In the second embodiment of the present invention, therefore, the user gains access to a more limited range of information regarding the development or conversion of plastic blood collection tubes. This embodiment can be better suited for applications in which a single user has entered a confidentiality agreement with a single information provider for access to a very narrow field of information or operations.

It will be appreciated that the development and conversion information and algorithms will be continuously upgraded, new information added and older information deleted as the technology develops, and additional tools applied in the extranet site screens can include the latest commercially available products. For example, Livelink Intranet available from Open Text Corp of Toronto, Ontario Canada can be used as a collaboration software application, and the Express and Trusted Link products from Harbinger Corp. of Atlanta, Ga. can be used for an electronic data interchange application software.

Although only certain exemplary embodiments of the present invention have been described in detail above, those skilled in the art will readily appreciate that many modifications are possible in the exemplary embodiments without materially departing from the novel teachings and advantages of the invention. Accordingly, all such modifications are intended to be included within the scope of the invention as defined in the appended claims and equivalents thereof.

## Claims

1. A method for providing an information exchange between a plurality of users (12,14,16,18) and an information provider (22) via a shared network (20), the method comprising:
the information provider (22) providing hardware and software support for the use of Internet protocols in a computer-based extranet site (30, 50) having a plurality of information levels (32,34,36,38,40,42), wherein each level of the plurality of information levels has a cumulative content so that each subsequent level includes new information, as well as all information disclosed in the earlier levels;
the shared network (20) providing access between the plurality of users and between the the plurality of users and the information provider, wherein access to the shared network is controlled through the use of confidentiality agreements between the plurality of users and between the plurality of users and the information provider;
the information provider (22) providing access between at least one user of said plurality of users and at least one level of said plurality of information levels within the information provider (22) based on a user specific access permission mechanism, said access comprising a computer display for providing an information exchange between said accessed at least one level and said at least one user, said display (44) comprising a work space notation, a list of categories for a selected work space (45), or a list of topics for a selected category (47); and
restricting access between said at least one user and one or more remaining levels of said plurality of information levels.

2. A method for providing an information exchange as claimed in claim 1, wherein said information exchange provides data and compliance support for use in at least one of a product development and a product conversion.

3. A method for providing an information exchange as claimed in claim 2, wherein said product comprises at least one of a plastic blood collection tube and a glass blood collection tube.

4. A method for providing an information exchange as claimed in Claim 2, wherein said accessed at least one level comprises:
a first level comprising of at least one of a manufacturer white paper published article and material related to at least one of said product development and said product conversion.

5. A method for providing an information exchange as claimed in claim 2, wherein said accessed at least one level comprises:
a second level including at least one of the contents of a first level and an analytical array database related to at least one of said product development and said product conversion.

6. A method for providing an information exchange as claimed in claim 2, wherein said accessed at least one level comprises:
a third level including at least one of the contents of a first and second level and a statistical equivalency software algorithm related to at least one of said product development and said product conversion.

7. A method for providing an information exchange as claimed in claim 2, wherein said accessed at least one level comprises:
a fourth level including at least one of the contents of a first, second, and third level and analytical advantage services related to at least one of said product development and said product conversion.

8. A method for providing an information exchange as claimed in claim 2, wherein said accessed at least one level comprises:
a fifth level including at least one of the contents of a first, second, third, and fourth level and at least one Internet link related to at least one of said product development and said product conversion.

9. A method for providing an information exchange as claimed in claim 1, wherein said providing access based on an access permission mechanism comprises:
querying a user of the plurality of users for a password in response to a request for access;
directing said user to a level based upon an access permission associated with said user; and
providing access between said user and at least one display of information.

10. A method for providing an information exchange as claimed in claim 9, wherein said access permission associated with said user is established by an agreement between said user and at least one of said provider and a second user of the plurality of users.

11. A method for providing an information exchange as claimed in claim 10, wherein said providing access is further restricted to exclude at least one element of said accessed at least one level based upon said access permission associated with said user.

12. An information exchange system to provide information exchanges between a plurality of users (12,14,16,18) and an information provider (22) via a shared network (20), comprising:
hardware and software apparatus of the information provider (22) for the use of Internet protocols at a computer-based extranet site (30, 50) having a plurality of information levels (32,34,36,38,40,42), wherein each level of the plurality of information levels has a cumulative content so that each subsequent level includes new information, as well as all information disclosed in the earlier levels;
a shared network (20) providing access between the plurality of users and between the plurality of users and the information provider, wherein access to the shared network (20) is controlled through the use of confidentiality agreements between the plurality of users and between the plurality of users and the information provider;
a user specific access permission system of the information provider (22) providing an access means between at least one user of said plurality of users and at least one level of said plurality of information levels within the information provider (22), said access means comprising a computer display for providing an information exchange between said accessed at least one level and said at least one user, said display (44) comprising a work space notation, a list of categories for a selected work space (45), or a list of topics for a selected category (47); and
an access restriction system restricting access between said at least one user and one or more remaining levels of said plurality of information levels.

13. An information exchange system as claimed in claim 12, wherein said information exchange comprises at least one of data and compliance support for use in at least one of a product development and a product conversion.

14. An information exchange system as claimed in claim 13, wherein said product comprises at least one of a plastic blood collection tube and a glass blood collection tube.

15. An information exchange system as claimed in claim 13, wherein said accessed at least one level comprises:
a first level of at least one of a manufacturer white paper published article and material related to at least one of said product development and said product conversion.

16. An information exchange system as claimed in claim 13, wherein said accessed at least one level comprises:
a second level including at least one of the contents of a first level and an analytical array database related to at least one of said product development and said product conversion.

17. An information exchange system as claimed in claim 13, wherein said accessed at least one level comprises:
a third level including at least one of the contents of a first and second level and a statistical equivalency software algorithm related to at least one of said product development and said product conversion.

18. An information exchange system as claimed in claim 13, wherein said accessed at least one level comprises:
a fourth level including at least one of the contents of a first, second, and third level and analytical advantage services related to at least one of said product development and said product conversion.

19. An information exchange system as claimed in claim 13, wherein said accessed at least one level comprises:
a fifth level including at least one of the contents of a first, second, third, and fourth level and at least one internet link related to at least one of said product development and said product conversion.

20. An information exchange system as claimed in claim 12, wherein said access permission system comprises:
a query display adapted to request a user of the plurality of users for password in response to a request for access;
a first routing system for directing said user to a level based upon an access permission associated with said user; and
a second routing system providing access between said user and at least one of a display related to at least one of said product development and said product conversion.

21. An information exchange system as claimed in claim 20, wherein said access permission associated with said user is established by an agreement between said user and at least one of said provider and a second user of the plurality of users.

22. An information exchange system as claimed in claim 20, wherein said access restriction system comprises:
a third routing system for preventing said user from accessing a level based upon said access permission associated with said user.

23. An information exchange system as claimed in claim 20, wherein said access restriction system further comprises:
a fourth routing system for excluding at least one element of said accessed level based upon said access permission associated with said user.

## Patentansprüche

1. Verfahren zum Bereitstellen eines Informationsaustauschs zwischen einer Vielzahl von Nutzern (12, 14, 16, 18) und einem Informations-Provider (22) über ein gemeinsam genutztes Netzwerk (20), wobei das Verfahren umfasst:
Bereitstellen, durch den Informations-Provider (22), von Hardware- und Software-Support für die Verwendung von Internetprotokollen auf einer computerbasierten Extranet-Site (30, 50) mit einer Vielzahl von Informationslevels (32, 34, 36, 38, 40, 42), wobei jeder Level der Vielzahl von Informationslevels einen kumulativen Inhalt aufweist, so dass jeder nachfolgende Level neue Informationen sowie sämtliche Informationen, die in den vorhergehenden Levels offengelegt worden sind, enthält;
Bereitstellen, durch das gemeinsam genutzte Netzwerk (20), von Zugriff zwischen der Vielzahl von Nutzern und zwischen der Vielzahl von Nutzern und dem Informations-Provider, wobei der Zugriff auf das gemeinsam genutzte Netzwerk durch die Verwendung von Geheimhaltungsvereinbarungen zwischen der Vielzahl von Nutzern und zwischen der Vielzahl von Nutzern und dem Informations-Provider gesteuert wird;
Bereitstellen, durch den Informations-Provider (22), von Zugriff zwischen mindestens einem Nutzer der Vielzahl von Nutzern und mindestens einem Level der Vielzahl von Informationslevels innerhalb des Informations-Providers (22) auf der Basis eines nutzerspezifischen Zugriffserlaubnismechanismus, wobei der Zugriff ein Computer-Display zum Bereitstellen eines Informationsaustauschs zwischen dem mindestens einen Level, auf den zugegriffen wird, und dem mindestens einen Nutzer umfasst, wobei das Display (44) eine Arbeitsraumnotation, eine Liste von Kategorien für einen ausgewählten Arbeitsraum (45) oder eine Liste von Themen für eine ausgewählte Kategorie (47) aufweist; und
Einschränken von Zugriff zwischen dem mindestens einen Nutzer und einem oder mehreren verbliebenen Levels der Vielzahl von Informationslevels.

2. Verfahren zum Bereitstellen eines Informationsaustauschs nach Anspruch 1, bei dem beim Informationsaustausch Daten und Compliance-Support zur Verwendung bei einer Produktentwicklung und/oder einer Produktumstellung bereitgestellt werden.

3. Verfahren zum Bereitstellen eines Informationsaustauschs nach Anspruch 2, bei dem das Produkt ein Kunststoff-Blutaufnahmeröhrchen und/oder ein Glas-Blutaufnahmeröhrchen umfasst.

4. Verfahren zum Bereitstellen eines Informationsaustauschs nach Anspruch 2, bei dem der mindestens eine Level, auf den zugegriffen wird, umfasst:
einen ersten Level, der einen von einem Hersteller in einem White Paper veröffentlichten Artikel und/oder Material, das sich auf die Produktentwicklung und/oder die Produktumstellung bezieht, aufweist.

5. Verfahren zum Bereitstellen eines Informationsaustauschs nach Anspruch 2, bei dem der mindestens eine Level, auf den zugegriffen wird, umfasst:
einen zweiten Level, der den Inhalt des ersten Levels und/oder eine Analytisches-Array-Datenbank, die sich auf die Produktentwicklung und/oder die Produktumstellung bezieht, aufweist.

6. Verfahren zum Bereitstellen eines Informationsaustauschs nach Anspruch 2, bei dem der mindestens eine Level, auf den zugegriffen wird, umfasst:
einen dritten Level, der den Inhalt des ersten und des zweiten Levels und/oder einen Statistische-Äquivalenz-Software-Algorithmus, der sich auf die Produktentwicklung und/oder die Produktumstellung bezieht, aufweist.

7. Verfahren zum Bereitstellen eines Informationsaustauschs nach Anspruch 2, bei dem der mindestens eine Level, auf den zugegriffen wird, umfasst:
einen vierten Level, der den Inhalt eines ersten, zweiten und dritten Levels und/oder Analytischer-Vorteil-Dienste, die sich auf die Produktentwicklung und/oder die Produktumstellung beziehen, aufweist.

8. Verfahren zum Bereitstellen eines Informationsaustauschs nach Anspruch 2, bei dem der mindestens eine Level, auf den zugegriffen wird, umfasst:
einen fünften Level, der den Inhalt des ersten, zweiten, dritten und vierten Levels und/oder mindestens einen Internetlink, der sich auf die Produktentwicklung und/oder die Produktumstellung bezieht, aufweist.

9. Verfahren zum Bereitstellen eines Informationsaustauschs nach Anspruch 1, bei dem das Bereitstellen von Zugriff auf der Basis eines Zugriffserlaubnismechanismus umfasst:
Befragen eines Nutzers der Vielzahl von Nutzern hinsichtlich eines Passworts in Reaktion auf eine Zugriffsanforderung;
Leiten des Nutzers zu einem Level auf der Basis einer dem Nutzer zugeordneten Zugriffserlaubnis; und
Bereitstellen von Zugriff zwischen dem Nutzer und mindestens einem Informations-Display.

10. Verfahren zum Bereitstellen eines Informationsaustauschs nach Anspruch 9, bei dem die einem Nutzer zugeordnete Zugriffserlaubnis mittels einer Vereinbarung zwischen dem Nutzer und dem Provider und/oder einem zweiten Nutzer der Vielzahl von Nutzern erstellt wird.

11. Verfahren zum Bereitstellen eines Informationsaustauschs nach Anspruch 10, bei dem das Bereitstellen von Zugriff ferner eingeschränkt ist, um mindestens ein Element des mindestens einen Levels, auf den zugegriffen wird, auf der Basis der dem Nutzer zugeordneten Zugriffserlaubnis auszuschließen.

12. Informationsaustauschsystem zum Bereitstellen eines Informationsaustausches zwischen einer Vielzahl von Nutzern (12, 14, 16, 18) und einem Informations-Provider (22) über ein gemeinsam genutztes Netzwerk (20), umfassend:
Hardware- und Software-Vorrichtungen des Informations-Providers (22) für die Verwendung von Internetprotokollen auf einer computerbasierten Extranet-Site (30, 50) mit einer Vielzahl von Informationslevels (32, 34, 36, 38, 40, 42), wobei jeder Level der Vielzahl von Informationslevels einen kumulativen Inhalt aufweist, so dass jeder nachfolgende Level neue Informationen sowie sämtliche Informationen, die in den vorhergehenden Levels offengelegt worden sind, enthält;
ein gemeinsam genutztes Netzwerk (20), das Zugriff zwischen der Vielzahl von Nutzern und zwischen der Vielzahl von Nutzern und dem Informations-Provider bereitstellt, wobei der Zugriff auf das gemeinsam genutzte Netzwerk (20) durch die Verwendung von Geheimhaltungsvereinbarungen zwischen der Vielzahl von Nutzern und zwischen der Vielzahl von Nutzern und dem Informations-Provider gesteuert wird;
ein nutzerspezifisches Zugriffserlaubnissystem des Informations-Providers (22), das eine Zugriffseinrichtung zwischen mindestens einem Nutzer der Vielzahl von Nutzern und mindestens einem Level der Vielzahl von Informationslevels innerhalb des Informations-Providers (22) bereitstellt, wobei die Zugriffseinrichtung ein Computer-Display zum Bereitstellen eines Informationsaustauschs zwischen dem mindestens einen Level, auf den zugegriffen wird, und dem mindestens einen Nutzer aufweist, wobei das Display (44) eine Arbeitsraumnotation, eine Liste von Kategorien für einen ausgewählten Arbeitsraum (45) oder eine Liste von Themen für eine ausgewählte Kategorie (47) aufweist; und
ein Zugriffseinschränkungssystem zum Einschränken von Zugriff zwischen dem mindestens einen Nutzer und einem oder mehreren verbliebenen Levels der Vielzahl von Informationslevels.

13. Informationsaustauschsystem nach Anspruch 12, bei dem der Informationsaustausch Daten und/oder Compliance-Support zur Verwendung bei einer Produktentwicklung und/oder einer Produktumstellung umfasst.

14. Informationsaustauschsystem nach Anspruch 13, bei dem das Produkt ein Kunststoff-Blutaufnahmeröhrchen und/oder ein Glas-Blutaufnahmeröhrchen umfasst.

15. Informationsaustauschsystem nach Anspruch 13, bei dem der mindestens eine Level, auf den zugegriffen wird, umfasst:
einen ersten Level eines von einem Hersteller in einem White Paper veröffentlichten Artikels und/oder Material, das sich auf die Produktentwicklung und/oder die Produktumstellung bezieht.

16. Informationsaustauschsystem nach Anspruch 13, bei dem der mindestens eine Level, auf den zugegriffen wird, umfasst:
einen zweiten Level, der den Inhalt des ersten Levels und/oder eine Analytisches-Array-Datenbank, die sich auf die Produktentwicklung und/oder die Produktumstellung bezieht, aufweist.

17. Informationsaustauschsystem nach Anspruch 13, bei dem der mindestens eine Level, auf den zugegriffen wird, umfasst:
einen dritten Level, der den Inhalt des ersten und des zweiten Levels und/oder einen Statistische-Äquivalenz-Software-Algorithmus, der sich auf die Produktentwicklung und/oder die Produktumstellung bezieht, aufweist.

18. Informationsaustauschsystem nach Anspruch 13, bei dem der mindestens eine Level, auf den zugegriffen wird, umfasst:
einen vierten Level, der den Inhalt eines ersten, zweiten und dritten Levels und/oder Analytischer-Vorteil-Dienste, die sich auf die Produktentwicklung und/oder die Produktumstellung beziehen, aufweist.

19. Informationsaustauschsystem nach Anspruch 13, bei dem der mindestens eine Level, auf den zugegriffen wird, umfasst:
einen fünften Level, der den Inhalt des ersten, zweiten, dritten und vierten Levels und/oder mindestens einen Internetlink, der sich auf die Produktentwicklung und/oder die Produktumstellung bezieht, aufweist.

20. Informationsaustauschsystem nach Anspruch 12, bei dem das Zugriffserlaubnissystem umfasst:
ein Befragungs-Display, das derart ausgebildet ist, dass es von einem Nutzer der Vielzahl von Nutzern ein Passwort in Reaktion auf eine Zugriffsanforderung anfordert;
ein erstes Routing-System zum Leiten des Nutzers zu einem Level auf der Basis einer dem Nutzer zugeordneten Zugriffserlaubnis; und
ein zweites Routing-System zum Bereitstellen von Zugriff zwischen dem Nutzer und mindestens einem Display, das sich auf die Produktentwicklung und/oder die Produktumstellung bezieht.

21. Informationsaustauschsystem nach Anspruch 20, bei dem die einem Nutzer zugeordnete Zugriffserlaubnis mittels einer Vereinbarung zwischen dem Nutzer und dem Provider und/oder einem zweiten Nutzer der Vielzahl von Nutzern erstellt wird.

22. Informationsaustauschsystem nach Anspruch 20, bei dem das Zugriffseinschränkungssystem umfasst:
ein drittes Routing-System zum Verhindern, dass der Benutzer auf einen Level auf der Basis der dem Nutzer zugeordneten Zugriffserlaubnis zugreift.

23. Informationsaustauschsystem nach Anspruch 20, bei dem das Zugriffseinschränkungssystem umfasst:
ein viertes Routing-System zum Ausschließen mindestens eines Elements des Levels, auf den zugegriffen wird, auf der Basis der dem Nutzer zugeordneten Zugriffserlaubnis.

## Revendications

1. Procédé pour fournir un échange d'informations entre une pluralité d'utilisateurs (12, 14, 16, 18) et un fournisseur d'informations (22) via un réseau partagé (20), le procédé comprenant le fait :
de fournir, par le biais du fournisseur d'informations (22), un support matériel et logiciel pour l'utilisation de protocoles Internet dans un site extranet informatisé (30, 50) ayant une pluralité de niveaux d'informations (32, 34, 36, 38, 40, 42), où chaque niveau de la pluralité de niveaux d'informations a un contenu cumulatif de sorte que chaque niveau suivant comporte de nouvelles informations, ainsi que toutes les informations divulguées dans les niveaux précédents ;
de fournir, par le biais du réseau partagé (20), un accès entre la pluralité d'utilisateurs et entre la pluralité d'utilisateurs et le fournisseur d'informations, où l'accès au réseau partagé est contrôlé par l'utilisation d'accords de confidentialité entre la pluralité d'utilisateurs et entre la pluralité d'utilisateurs et le fournisseur d'informations ;
de fournir, par le biais du fournisseur d'informations (22), un accès entre au moins un utilisateur de ladite pluralité d'utilisateurs et au moins un niveau de ladite pluralité de niveaux d'informations au sein du fournisseur d'informations (22) sur la base d'un mécanisme d'autorisation d'accès spécifique à l'utilisateur, ledit accès comprenant un dispositif d'affichage informatique pour fournir un échange d'informations entre ledit au moins un niveau auquel on accède et ledit au moins un utilisateur, ledit dispositif d'affichage (44) comprenant une notation d'espace de travail, une liste de catégories pour un espace de travail sélectionné (45), ou une liste de sujets pour une catégorie sélectionnée (47) ; et
de limiter l'accès entre ledit au moins un utilisateur et un ou plusieurs niveau(x) restant(s) de ladite pluralité de niveaux d'informations.

2. Procédé pour fournir un échange d'informations selon la revendication 1, dans lequel ledit échange d'informations fournit des données et un support de conformité à utiliser dans au moins l'un(e) d'un développement de produit et d'une conversion de produit.

3. Procédé pour fournir un échange d'informations selon la revendication 2, dans lequel ledit produit comprend au moins l'un d'un tube de prélèvement de sang en plastique et d'un tube de prélèvement de sang en verre.

4. Procédé pour fournir un échange d'informations selon la revendication 2, dans lequel ledit au moins un niveau auquel on accède comprend :
un premier niveau comprenant au moins l'un d'un article publié de document de présentation technique de fabricant et d'un matériau lié à au moins l'un(e) dudit développement de produit et de ladite conversion de produit.

5. Procédé pour fournir un échange d'informations selon la revendication 2, dans lequel ledit au moins un niveau auquel on accède comprend :
un deuxième niveau comportant au moins l'un des contenus d'un premier niveau et une base de données de réseau analytique liée à au moins l'un(e) dudit développement de produit et de ladite conversion de produit.

6. Procédé pour fournir un échange d'informations selon la revendication 2, dans lequel ledit au moins un niveau auquel on accède comprend :
un troisième niveau comportant au moins l'un des contenus d'un premier et d'un deuxième niveau et un algorithme de logiciel d'équivalence statistique lié à au moins l'un(e) dudit développement de produit et de ladite conversion de produit.

7. Procédé pour fournir un échange d'informations selon la revendication 2, dans lequel ledit au moins un niveau auquel on accède comprend :
un quatrième niveau comportant au moins l'un des contenus d'un premier, deuxième et troisième niveau et des services d'avantage analytique liés à au moins l'un(e) dudit développement de produit et de ladite conversion de produit.

8. Procédé pour fournir un échange d'informations selon la revendication 2, dans lequel ledit au moins un niveau auquel on accède comprend :
un cinquième niveau comportant au moins l'un des contenus d'un premier, deuxième, troisième et quatrième niveau et au moins un lien Internet lié à au moins l'un(e) dudit développement de produit et de ladite conversion de produit.

9. Procédé pour fournir un échange d'informations selon la revendication 1, dans lequel ladite fourniture d'accès sur la base d'un mécanisme d'autorisation d'accès comprend le fait :
d'interroger un utilisateur de la pluralité d'utilisateurs sur un mot de passe en réponse à une demande d'accès ;
de diriger ledit utilisateur vers un niveau sur la base d'une autorisation d'accès associée audit utilisateur ; et
de fournir un accès entre ledit utilisateur et au moins un dispositif d'affichage d'informations.

10. Procédé pour fournir un échange d'informations selon la revendication 9, dans lequel ladite autorisation d'accès associée audit utilisateur est établie par un accord entre ledit utilisateur et au moins l'un dudit fournisseur et d'un deuxième utilisateur de la pluralité d'utilisateurs.

11. Procédé pour fournir un échange d'informations selon la revendication 10, dans lequel ladite fourniture d'accès est en outre limitée pour exclure au moins un élément dudit au moins un niveau auquel on accède sur la base de ladite autorisation d'accès associée audit utilisateur.

12. Système d'échange d'informations pour fournir des échanges d'informations entre une pluralité d'utilisateurs (12, 14, 16, 18) et un fournisseur d'informations (22) via un réseau partagé (20), comprenant :
un appareil matériel et logiciel du fournisseur d'informations (22) pour l'utilisation de protocoles Internet sur un site extranet informatisé (30, 50) ayant une pluralité de niveaux d'informations (32, 34, 36, 38, 40, 42), dans lequel chaque niveau de la pluralité de niveaux d'informations a un contenu cumulatif de sorte que chaque niveau suivant comporte de nouvelles informations, ainsi que toutes les informations divulguées dans les niveaux précédents ;
un réseau partagé (20) fournissant un accès entre la pluralité d'utilisateurs et entre la pluralité d'utilisateurs et le fournisseur d'informations, où l'accès au réseau partagé (20) est contrôlé par l'utilisation d'accords de confidentialité entre la pluralité d'utilisateurs et entre la pluralité d'utilisateurs et le fournisseur d'informations ;
un système d'autorisation d'accès spécifique à l'utilisateur du fournisseur d'informations (22) fournissant un moyen d'accès entre au moins un utilisateur de ladite pluralité d'utilisateurs et au moins un niveau de ladite pluralité de niveaux d'informations au sein du fournisseur d'informations (22), ledit moyen d'accès comprenant un dispositif d'affichage informatique pour fournir un échange d'informations entre ledit au moins un niveau auquel on accède et ledit au moins un utilisateur, ledit dispositif d'affichage (44) comprenant une notation d'espace de travail, une liste de catégories pour un espace de travail sélectionné (45), ou une liste de sujets pour une catégorie sélectionnée (47) ; et
un système de restriction d'accès limitant l'accès entre ledit au moins un utilisateur et un ou plusieurs niveau(x) restant(s) de ladite pluralité de niveaux d'informations.

13. Système d'échange d'informations selon la revendication 12, dans lequel ledit échange d'informations comprend des données et/ou un support de conformité à utiliser dans au moins l'un(e) d'un développement de produit et d'une conversion de produit.

14. Système d'échange d'informations selon la revendication 13, dans lequel ledit produit comprend au moins l'un d'un tube de prélèvement de sang en plastique et d'un tube de prélèvement de sang en verre.

15. Système d'échange d'informations selon la revendication 13, dans lequel ledit au moins un niveau auquel on accède comprend :
un premier niveau d'au moins l'un d'un article publié de document de présentation technique de fabricant et d'un matériau lié à au moins l'un dudit développement de produit et de ladite conversion de produit.

16. Système d'échange d'informations selon la revendication 13, dans lequel ledit au moins un niveau auquel on accède comprend :
un deuxième niveau comportant au moins l'un des contenus d'un premier niveau et une base de données de réseau analytique liée à au moins l'un(e) dudit développement de produit et de ladite conversion de produit.

17. Système d'échange d'informations selon la revendication 13, dans lequel ledit au moins un niveau auquel on accède comprend :
un troisième niveau comportant au moins l'un des contenus d'un premier et d'un deuxième niveau et un algorithme de logiciel d'équivalence statistique lié à au moins l'un(e) dudit développement de produit et de ladite conversion de produit.

18. Système d'échange d'informations selon la revendication 13, dans lequel ledit au moins un niveau auquel on accède comprend :
un quatrième niveau comportant au moins l'un des contenus d'un premier, deuxième et troisième niveau et des services d'avantage analytique liés à au moins l'un(e) dudit développement de produit et de ladite conversion de produit.

19. Système d'échange d'informations selon la revendication 13, dans lequel ledit au moins un niveau auquel on accède comprend :
un cinquième niveau comportant au moins l'un des contenus d'un premier, deuxième, troisième et quatrième niveau et au moins un lien Internet lié à au moins l'un(e) dudit développement de produit et de ladite conversion de produit.

20. Système d'échange d'informations selon la revendication 12, dans lequel ledit système d'autorisation d'accès comprend :
un dispositif d'affichage d'interrogation adapté pour demander à un utilisateur de la pluralité d'utilisateurs un mot de passe en réponse à une demande d'accès ;
un premier système de routage pour diriger ledit utilisateur vers un niveau sur la base d'une autorisation d'accès associée audit utilisateur ; et
un deuxième système de routage fournissant un accès entre ledit utilisateur et au moins un affichage lié à au moins l'un(e) dudit développement de produit et de ladite conversion de produit.

21. Système d'échange d'informations selon la revendication 20, dans lequel ladite autorisation d'accès associée audit utilisateur est établie par un accord entre ledit utilisateur et au moins l'un desdits fournisseurs et un deuxième utilisateur de la pluralité d'utilisateurs.

22. Système d'échange d'informations selon la revendication 20, dans lequel ledit système de restriction d'accès comprend :
un troisième système de routage pour empêcher ledit utilisateur d'accéder à un niveau sur la base de ladite autorisation d'accès associée audit utilisateur.

23. Système d'échange d'informations selon la revendication 20, dans lequel ledit système de restriction d'accès comprend en outre :
un quatrième système de routage pour exclure au moins un élément dudit niveau auquel on accède sur la base de ladite autorisation d'accès associée audit utilisateur.
